# EUROPEAN PATENT APPLICATION

(11) **EP 4 009 203 A1**
(43) Date of publication of application: **08.06.2022**
(21) Application number: 20849566.3
(22) Date of filing: 03.08.2020
(51) Int. Cl.: G06F 21/32, G06F 21/44, G06Q 50/30, A61B 5/1171

(54) **BIOLOGICAL INFORMATION MEASUREMENT SYSTEM, FRAUD MEASUREMENT DETERMINATION METHOD, FRAUD MEASUREMENT DETERMINATION PROGRAM, AND NON-TRANSITORY COMPUTER-READABLE RECORDING MEDIUM**

(30) Priority: 02.08.2019 JP 2019142803
(71) Applicant: Tanita Corporation, Tokyo 174-8630 (JP)
(72) Inventor: MOCHIZUKI Kei, Tokyo 174-8630 (JP); SAGAWA Kiyoshi, Tokyo 174-8630 (JP); KASAHARA Yasuhiro, Tokyo 174-8630 (JP); IKEDA Satoshi, Tokyo 174-8630 (JP)
(74) Representative: Eisenführ Speiser
(86) International application number: PCT/JP2020/029630
(87) International publication number: WO 2021/024974

(57) **Abstract**

In an alcohol detection system (10), an authentication server (16) receives camera image information acquired by a camera (32) shooting an image of an indication ID displayed on a display (22) of an alcohol detection device (12) and a device ID sent from the alcohol detection device (12). The authentication server (16) then determines whether a person to be measured is performing alcohol detection using the alcohol detection device (12) an image of which has been shot by the camera (32) or not based on the indication ID indicated by the camera image information and the device ID sent from the alcohol detection device (12).

## Description

### CROSS-REFERENCE TO RELATED APPLICATIONS

This application claims the benefit of Japanese Patent Application No. 2019-142803 filed on August 2, 2019 in Japan, the contents of which are incorporated herein by reference.

### TECHNICAL FIELD

The present invention relates to a biological information measuring system, a fraudulent-measurement determination method, a fraudulent-measurement determination program, and a computer-readable non-transitory storage medium.

### BACKGROUND ART

Alcohol testing is recently widely performed on drivers and crew members of business vehicles, aircrafts, ships, railroad trains, or the like by using alcohol detectors at roll call or the like. When an alcohol test is performed, frauds are prevented generally by an onsite checker (an observer) checking a driver or a crew member, who is a person to be measured, against a photograph of the face of the person to be measured during alcohol measurement done by the person to be measured. Roll call, however, is sometimes performed remotely in such a way that a driver or a crew member is in a remote location and a checker does the check via a monitor or the like. In such a case, a tablet terminal or another terminal device is used to send a photograph of the face of a person to be measured themself, and therefore frauds may be committed that include measurement performed by another person impersonating the person to be measured and measurement with a different device (a dummy device).

In this regard, Patent document 1 (Japanese Patent Laid-Open Application No. 2013-192859) includes a biological information measuring system comprising: a biological information measuring device provided with a light-emitting unit; a first terminal device provided with a camera; and a second terminal device for face recognition processing to authenticate a person or the like, for the purpose of effectively preventing fraudulent measurement using a dummy device and another person than a person to be measured.

In this biological information measuring system, a measurer operates an input unit of the second terminal device to cause a light emission instruction signal indicating a light emission pattern, that the second terminal device has been caused to determine, to be sent from the second terminal device to the biological measuring device before measurement with the biological information measuring device is started. The camera then keeps on shooting the light-emitting unit emitting light according to the light emission pattern and the face of a person to be measured, and stores the shot data in a storage of the first terminal device. The second terminal device determines whether the light emitted from the light-emitting unit visible in the shot data shot by the camera is in accordance with the light emission pattern or not, and determines whether face data generated from the shot data and a registered face image represent the face of the same person or not.

As a result, this biological information measuring system can determine that the biological information measuring device shot by the camera is the very biological information measuring device used for the measurement and that the person to be measured shot by the camera is the very person, and therefore a fraudulent measurement using a so-called substitute can be prevented effectively.

### SUMMARY OF THE INVENTION

### Problems to be solved by the invention

The biological information measuring system disclosed in Patent document 1, however, involves troublesome exchanges of information in the system since the light emission instruction signal is sent and received between the second terminal device and the biological information measuring device. In addition, if the light emission pattern formed by the light emission instruction signal is simple, a fraudulent measurement can be done in such a way that using another biological information measuring device to reproduce the light emission pattern and shooting the light with a camera cause the system to determine as if a person to be measured were performing the measurement with the biological information measuring device that received the light emission instruction signal even though the measurement is being performed with the other biological information measuring device.

A purpose of the disclosure made in view of the above is to provide a biological information measuring system, a fraudulent-measurement determination method, a fraudulent-measurement determination program, and a computer-readable non-transitory storage medium that can determine whether a person to be measured themself is measuring biological information using a biological information measuring device or not with simpler components.

### Means for solving the problems

A biological information measuring system of an aspect of the disclosure comprises: a biological information measuring device for measuring biological information of a person to be measured; an image-shooting device; and an information-processing device, where the biological information measuring device comprises: display means for displaying indication identification information; and sending means for sending device identification information, and where the information-processing device comprises: receiving means for receiving image information acquired by the image-shooting device shooting an image of the indication identification information displayed on the display means and the device identification information sent from the sending means; and determination means for determining whether the person to be measured is measuring the biological information using the biological information measuring device an image of which has been shot by the image-shooting device or not based on the indication identification information indicated by the image information and the device identification information sent from the sending means.

A biological information measuring device and an information-processing device in a conventional art would send and receive identification information between each other in such a way that the information-processing device sends identification information to the biological information measuring device, which sends (sends back) the identification information to the information-processing device. In the present configuration, on the other hand, the transmission of identification information (the indication identification information and the device identification information) for use in authenticating the biological information measuring device is one way, that is, from the biological information measuring device to the information-processing device. Accordingly, the configuration simplifies the exchange of information used for identifying a person to be measured in the system as compared to the conventional art, and therefore allows determination of whether the person to be measured themself is measuring biological information using the biological information measuring device or not with simpler components.

In the above-described biological information measuring system, the biological information of the person to be measured may be breath alcohol concentration of the person to be measured.

This configuration allows determination of whether the measurement is performed by the person to be measured themself who performs alcohol detection or not with simpler components.

In the above-described biological information measuring system, the indication identification information may be randomly generated by the biological information measuring device each time the biological information is measured.

Since it is difficult to predict a random number value, this configuration allows determination of whether the biological information measuring device an image of which has been shot is the very device used for measuring the biological information of the person to be measured or not with simpler components.

In the above-described biological information measuring system, the indication identification information may be a value indicating a measurement result obtained by the biological information measuring device.

Since a measurement result is generated for each measurement, this configuration allows determination of whether the biological information measuring device an image of which has been shot is the very device used for measuring the biological information of the person to be measured or not with simpler components.

In the above-described biological information measuring system, the image-shooting device may shoot an image of a face of the person to be measured who is performing measurement with the biological information measuring device as well as the image of the indication identification information displayed on the display means, and the information-processing device may comprise face authentication means for performing face authentication on the person to be measured based on face image information that is indicated by the image information and indicates the face of the person to be measured.

This configuration allows face authentication to be performed along with the authentication using the indication identification information and the device identification information, and therefore allows a more reliable determination of whether the measurement is performed by the person to be measured themself or not.

In the above-described biological information measuring system, the face authentication means may perform the face authentication based on a plurality of pieces of face image information, images of which have been shot continuously.

This configuration allows the face authentication to be performed by using a plurality of pieces of face image information, and therefore allows a more reliable determination of whether the measurement is performed by the person to be measured themself or not.

In the above-described biological information measuring system, the face authentication means may perform face authentication on the person to be measured with the face image information and registered face image information that is registered in advance and indicates the face of the person to be measured.

This configuration allows face authentication to be performed by using registered face image information that is registered in advance, and therefore allows a more reliable determination of whether the measurement is performed by the person to be measured themself or not.

The above-described biological information measuring system may comprise body movement determination means for determining whether the person to be measured is moving or not based on the image information.

This configuration allows determination of whether the person to be measured who is being shot is a living body or not, and therefore allows a more reliable determination of whether the measurement is performed by the person to be measured themself or not.

In the above-described biological information measuring system, each time the biological information is measured, the biological information measuring device may send measurement performance information that is information on how the measurement of the biological information is performed to the information-processing device, and the information-processing device may comprise performance status determination means for determining whether the biological information measuring device is properly measuring the biological information or not based on the measurement performance information sent from the biological information measuring device.

This configuration prevents fraudulent acts or the like such as a person to be measured stopping the measurement of the biological information in the middle or disrupting communication between the biological information measuring device and the information-processing device, and allows determination of whether the measurement has been properly performed by the biological information measuring device or not.

In the above-described biological information measuring system, the biological information of the person to be measured may be measured during a predetermined time period.

This configuration allows the biological information of the person to be measured to be measured around an intended time period.

In the above-described biological information measuring system, the person to be measured may be a crew member of an aircraft, and the predetermined time period may be at least one of specified time periods before and after an operation time period of the aircraft that the crew member boards for service.

This configuration allows the biological information of the crew member of the aircraft to be measured around the operation time of the aircraft.

In the above-described biological information measuring system, the biological information measuring device may comprise an accelerometer, and if the accelerometer detects an acceleration greater than or equal to a specified value while the biological information measuring device is performing the measurement, the determination means may determine that the person to be measured is not measuring the biological information using the biological information measuring device an image of which has been shot by the image-shooting device.

The accelerometer detecting an acceleration greater than or equal to a specified value while the biological information measuring device is performing the measurement means that the biological information measuring device is moved instantaneously, and the biological information measuring device may be handed from the person to be measured to another person. This configuration therefore allows determination of whether a fraudulent act has been committed or not.

A fraudulent-measurement determination method of an aspect of the disclosure is an authentication method for a biological information measuring system comprising: a biological information measuring device for measuring biological information of a person to be measured; an image-shooting device; and an information-processing device, and the fraudulent-measurement determination method has: a first step of displaying indication identification information on display means and sending device identification information to the information-processing device; a second step of the image-shooting device shooting an image of the indication identification information displayed on the display means; a third step of the information-processing device receiving image information acquired by the image-shooting device shooting the image and the device identification information sent from the biological information measuring device; and a fourth step of the information-processing device determining whether the person to be measured is measuring the biological information using the biological information measuring device an image of which has been shot by the image-shooting device or not based on the indication identification information indicated by the image information and the device identification information sent from the biological information measuring device.

A fraudulent-measurement determination program of an aspect of the disclosure is for a biological information measuring system comprising: a biological information measuring device for measuring biological information of a person to be measured; an image-shooting device; and an information-processing device, and the fraudulent-measurement determination program causes a computer to execute: a first step of displaying indication identification information on display means and sending device identification information to the information-processing device; a second step of the image-shooting device shooting an image of the indication identification information displayed on the display means; a third step of the information-processing device receiving image information acquired by the image-shooting device shooting the image and the device identification information sent from the biological information measuring device; and a fourth step of the information-processing device determining whether the person to be measured is measuring the biological information using the biological information measuring device an image of which has been shot by the image-shooting device or not based on the indication identification information indicated by the image information and the device identification information sent from the biological information measuring device.

### ADVANTAGE OF THE INVENTION

The present disclosure allows determination of whether a person to be measured themself is measuring biological information using a biological information measuring device or not with a simpler configuration.

### BRIEF DESCRIPTION OF THE DRAWINGS

Figure 1 is a schematic configuration view of an alcohol detection system of a first embodiment;
Figure 2 is a function block diagram of the alcohol detection system of the first embodiment;
Figure 3 is a schematic diagram showing alcohol detection time periods of the first embodiment;
Figure 4 is a flowchart of an alcohol detection process of the first embodiment;
Figure 5 is a flowchart of the alcohol detection process of the first embodiment;
Figure 6 is a function block diagram of an alcohol detection system of a second embodiment;
Figure 7 is a flowchart of an alcohol detection process of the second embodiment;
Figure 8 is a function block diagram of an alcohol detection system of a third embodiment;
Figure 9 is a flowchart of an alcohol detection process of the third embodiment; and
Figure 10 shows a schematic configuration of an alcohol detection system of another embodiment.

### MODES OF EMBODYING THE INVENTION

Embodiments will now be described with reference to the drawings. The embodiments described below are merely illustrative of ways to implement the disclosure, and do not limit the disclosure to the specific configurations described below. When the disclosure is to be implemented, any specific configuration may be appropriately adopted according to the embodiment.

### (First embodiment)

An alcohol detection system is taken as an example of the biological information measuring system in this embodiment. That is to say, the biological information is the breath alcohol concentration of a person to be measured and the biological information measuring device is an alcohol detection device in the embodiment. A pilot or a flight attendant, who is a crew member of an aircraft, is taken as an example of a person to be measured in the embodiment. Examples of the aircraft include, but are not limited to, a passenger plane and a cargo plane, and it may be a business jet or another aircraft.

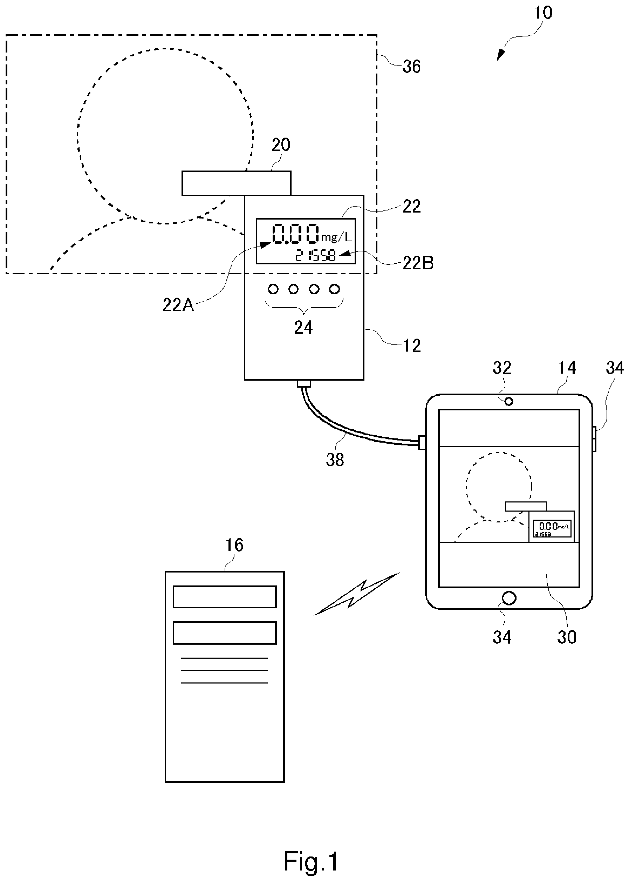

Figure 1 is a schematic configuration view of an alcohol detection system 10 of the embodiment. The alcohol detection system 10 comprises an alcohol detection device 12, a tablet terminal 14, and an authentication server 16. Note that each person to be measured has their own alcohol detection device 12 and tablet terminal 14 in the embodiment for example, but the embodiment is not limited to this, and a plurality of persons to be measured may share one alcohol detection device 12 with one another or a plurality of persons to be measured may share one tablet terminal 14 with one another.

The alcohol detection device 12 performs measurement of the alcohol concentration in the breath of a person to be measured (hereinafter also referred to as "alcohol detection"), and comprises a mouthpiece 20, a display 22, and an operation unit 24. The mouthpiece 20 is formed with an air inlet on one end and an air outlet on the other end. When a person to be measured holds the air inlet in the person's mouth and exhales into it, the alcohol concentration in the exhalation blown into the mouthpiece 20 is measured, and the blown breath is released from the air outlet.

The display 22 displays a result of the measurement of the alcohol concentration in the breath blown by a person to be measured (hereinafter referred to as an "alcohol measurement value") and the like. The operation unit 24 is operated by a person to be measured, and comprises a power button for powering on and off the alcohol detection device 12, a measurement start button for starting alcohol detection, a setup button for changing various settings and the like, and the like.

The alcohol detection device 12 of the embodiment generates an indication identification information (hereinafter referred to as an "indication ID"), and displays the indication ID on the display 22. An example of an indication ID of the embodiment is generated when the alcohol concentration is measured, and is displayed on the display 22. The alcohol detection device 12 of the embodiment has a measurement value display area 22A and an ID display area 22B in the display 22, and displays an alcohol measurement value in the measurement value display area 22A and an indication ID in the ID display area 22B.

The alcohol detection device 12 of the embodiment sends device identification information (hereinafter referred to as a "device ID") to the tablet terminal 14. An indication ID and a device ID of the embodiment are, for example, the same identification information, but the embodiment is not limited to this. An indication ID and a device ID do not have to be identical and are only required to have a correspondence relation that allows authentication based on the indication ID and the device ID as described in detail later.

The tablet terminal 14 is a portable information-processing device, and comprises a computer such as a CPU (Central Processing Unit) capable of executing a program, a storage device such as an SSD (Solid State Drive), a communication device for connecting to the Internet or an intranet, various types of connectors, a touch-panel display 30 for displaying an image and accepting an operation for the tablet terminal 14, a camera 32 for shooting an image of a subject, and an operation unit 34 for accepting an operation for the tablet terminal 14.

The tablet terminal 14 of the embodiment is held by a person to be measured who is a crew member of an aircraft. The tablet terminal 14, for example, holds a flight schedule of an aircraft that the crew member boards for service and is also used to manage the crew member's work. As described in detail later, a person to be measured performs alcohol detection based on a flight schedule.

The tablet terminal 14 receives an alcohol measurement value, a device ID, and the like sent from the alcohol detection device 12. When a person to be measured performs alcohol detection using the alcohol detection device 12, the camera 32 shoots an image so that the face of the person to be measured and the display 22 are included in the angle of view 36, and displays the shot image on the touch-panel display 30.

The tablet terminal 14 then sends the alcohol measurement value, the device ID, and image information acquired by the camera 32 shooting the image (hereinafter referred to as "camera image information") to the authentication server 16. The tablet terminal 14 also receives from the authentication server 16 a result of authentication based on the indication ID and the device ID, a result of authentication of the person to be measured based on the camera image information, and a result of determining whether a transport-related work can be performed or not based on alcohol detection (hereinafter referred to as a "transport-related work go/no-go result"), and displays them on the touch-panel display 30.

Note that the tablet terminal 14 is installed with an application software for performing an alcohol detection process using the alcohol detection device 12 (hereinafter referred to as the "alcohol detection app"). When performing alcohol detection, a person to be measured starts the alcohol detection app on the tablet terminal 14 and performs alcohol detection according to a procedure instructed by the alcohol detection app. In addition to the alcohol detection procedure, the alcohol detection app also cause an authentication result, a transport-related work go/no-go result, and the like received from the authentication server 16 to be displayed on the touch-panel display 30.

The authentication server 16 is an information-processing device comprising a computer such as a CPU capable of executing a program, a storage device such as an HDD (Hard Disk Drive), a communication device for connecting to the Internet or an intranet, various types of connectors, and the like. The authentication server 16 of the embodiment performs identification of a person to be measured and other various types of information processing. It holds pieces of information required for identification beforehand, and sequentially holds a measurement result (an alcohol measurement value) obtained by the alcohol detection device and the like in association with a person to be measured as described in detail later.

The authentication server 16 is connected to, for example, an information-processing device of a higher level for managing a crew member, a flight schedule, or the like (hereinafter referred to as a "management server"), an information-processing device used by a manager of a crew member (hereinafter referred to as a "management terminal"), or the like. This allows the authentication server 16 to send information based on alcohol detection to a management server or send to a predetermined mail address a result of an alcohol detection process described in detail later, or more specifically, failure in authentication or detection of a fraud. Upon viewing such a mail on a management terminal, a manager deals with the relevant crew member.

In the alcohol detection system 10 of the embodiment, the alcohol detection device 12 and the tablet terminal 14 send and receive information between each other via, for example, wire communication using a USB (Universal Serial Bus) cable 38. The communication method, however, is not limited to this, and information may be sent and received via, for example, Bluetooth (registered trademark) or other short-range wireless communication. The tablet terminal 14 and the authentication server 16 send and receive information between each other via, for example, the Internet or an intranet using Wi-Fi or other wireless communication.

Now, the alcohol detection system 10 of the embodiment makes identification for determining whether a person to be measured themself is performing alcohol detection using the alcohol detection device 12 or not. In other words, the alcohol detection system 10 of the embodiment prevents a person other than a person to be measured themself from pretending to be the person to be measured to fraudulently perform alcohol detection.

For this purpose, the authentication server 16 of the alcohol detection system 10 of the embodiment receives camera image information acquired by the camera 32 shooting an image of an indication ID displayed on the display 22 of the alcohol detection device 12 and a device ID sent from the alcohol detection device 12. The authentication server 16 then determines whether the person to be measured is performing alcohol detection using the alcohol detection device 12 an image of which has been shot by the camera 32 or not based on the indication ID indicated by the camera image information and the device ID sent from the alcohol detection device 12.

The transmission of IDs (the Indication ID and the device ID) for use in authentication in the alcohol detection system 10 of the embodiment is thus one way, that is, from the alcohol detection device 12 to the authentication server 16. Accordingly, the alcohol detection system 10 of the embodiment simplifies the exchange of information used for identifying a person to be measured in the system, and therefore allows determination of whether the person to be measured themself is performing alcohol detection using the alcohol detection device 12 or not with simpler components.

If the configuration were such that an ID is generated by the authentication server 16, is sent from the authentication server 16 to the alcohol detection device 12, and is displayed on the display 22 of the alcohol detection device 12 and if the ID were simple, a fraudulent measurement could be done in such a way that using another alcohol detection device 12 to reproduce the ID causes the system to determine as if the measurement is being performed with the alcohol detection device 12 that received the ID even though the measurement is being performed with the other alcohol detection device 12. A system in which an ID is sent only from the alcohol detection device 12 to the authentication server 16 like the alcohol detection device 12 of the embodiment, on the other hand, does not allow a fraud using two alcohol detection devices 12 described above, and can prevent such a fraud from occurring.

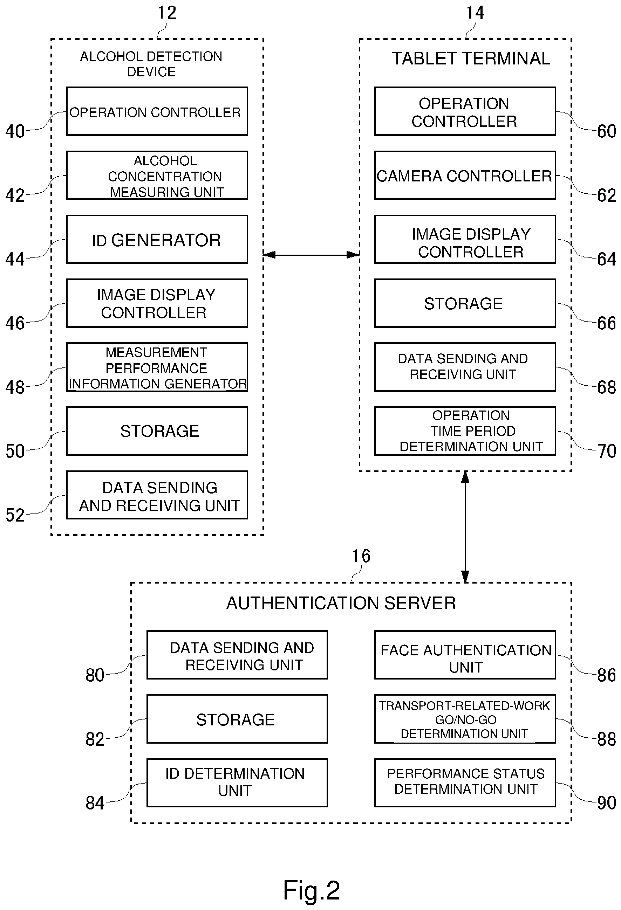

Figure 2 is a function block diagram for an alcohol detection process performed in the alcohol detection system 10 of the embodiment. Functions shown in Figure 2 in the embodiment are executed by, for example, computers each comprised in the alcohol detection device 12, the tablet terminal 14, and the authentication server 16. The configuration is not limited to this, and the functions may be implemented by ASICs (Application Specific Integrated Circuits) or other pieces of specific hardware each comprised in the alcohol detection device 12, the tablet terminal 14, and the authentication server 16.

The alcohol detection device 12 comprises an operation controller 40, an alcohol concentration measuring unit 42, an ID generator 44, an image display controller 46, a measurement performance information generator 48, a storage 50, and a data sending and receiving unit 52.

The operation controller 40 accepts an operation for the operation unit 24 done by a person to be measured, and provides an instruction for control according to the operation, such as on/off or the like of the alcohol detection device 12 or the start of alcohol detection.

The alcohol concentration measuring unit 42 measures the alcohol concentration in a breath blown through the mouthpiece 20 after the measurement start button included in the operation unit 24 is operated, and outputs the result as an alcohol measurement value.

The ID generator 44 generates an indication ID and a device ID each time alcohol concentration is measured. The ID generator 44 of the embodiment generates, for example, randomly generated pieces of information as an indication ID and a device ID. An examples of each randomly generated piece of information is a random number of a specified number of digits, and the random numbers may include numerics, characters, and symbols.

The image display controller 46 controls the display state of the display 22 in such a way as to cause an alcohol measurement value to be displayed in the measurement value display area 22A and cause an indication ID to be displayed in the ID display area 22B.

The measurement performance information generator 48 generates measurement performance information that is information on how alcohol detection is performed. Measurement performance information is, in other words, information that allows determination of how the alcohol detection device 12 is connected to the tablet terminal 14. The measurement performance information generator 48 of the embodiment counts the total number of alcohol detection performed by the alcohol detection device 12 (hereinafter referred to as the "total measurement number") as an example of measurement performance information. The total measurement number is an integrated value of the number of alcohol detection performed by the alcohol detection device 12 after shipment from the factory, and increases by one in value each time alcohol detection is performed.

The storage 50 holds indication and device IDs, the manufacturing ID of the alcohol detection device 12, the total measurement number, the setting status of the alcohol detection device 12, and the like. The manufacturing ID is an ID that is set at the time of manufacture of the alcohol detection device 12.

The data sending and receiving unit 52 sends and receives information to and from the tablet terminal 14 and another information-processing device through wire or wireless communication. The data sending and receiving unit 52 of the embodiment sends an alcohol measurement value, a device ID, the manufacturing ID, the total measurement number, and the like to the tablet terminal 14.

The tablet terminal 14 comprises an operation controller 60, a camera controller 62, an image display controller 64, a storage 66, a data sending and receiving unit 68, and an operation time period determination unit 70.

The operation controller 60 accepts an operation for the touch-panel display 30 and the operation unit 34 done by a person to be measured, and performs control according to the operation, such as execution of the alcohol detection app.

The camera controller 62 controls the camera 32 according to an operation done by a person to be measured.

The image display controller 64 controls the image display on the touch-panel display 30.

The storage 66 holds the alcohol detection app, camera image information, a flight schedule of a person to be measured who owns the tablet terminal 14, and the like. The flight schedule is sent from another information-processing device, and is automatically updated as required.

The data sending and receiving unit 68 sends and receives information to and from the alcohol detection device 12, the authentication server 16, and another information-processing device through wire or wireless communication. The data sending and receiving unit 68 of the embodiment receives an alcohol measurement value, a device ID, the manufacturing ID, and the total measurement number from the alcohol detection device 12, and sends these pieces of information to the authentication server 16 along with camera image information. The data sending and receiving unit 68 also receives an authentication result, a transport-related work go/no-go result, and the like from the authentication server 16.

The operation time period determination unit 70 determines a time period for alcohol detection based on a flight schedule stored in the storage 66. That is, the alcohol detection system 10 of the embodiment measures the alcohol concentration in the breath of a person to be measured in a predetermined time period. This allows the alcohol detection system 10 to measure the alcohol concentration of a person to be measured in an intended time period, that is, around the time when the person to be measured boards an aircraft for service in the embodiment.

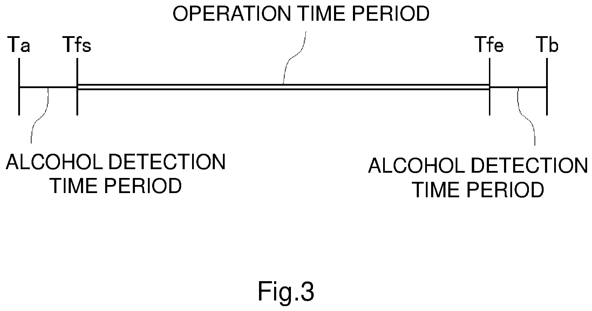

Figure 3 is a schematic diagram showing alcohol detection time periods of the embodiment. The operation time period shown in Figure 3 is an operation time of an aircraft that a person to be measured boards for service. In alcohol detection of the embodiment, a person to be measured performs alcohol detection within specified time periods before and after this operation time period.

In the example in Figure 3, time from a time Ta to an operation start time Tfs and time from an operation end time Tfe to a time Tb are set to be alcohol detection time periods. An interval between the operation start time Tfs and the time Ta and an interval between the operation end time Tfe and the time Tb are arbitrarily determined and are, for example, an hour. There may be a rule stating that a person to be measured should perform alcohol detection in at least one of time periods before and after an operation time period of an aircraft that the person to be measured boards for service. For example, the rule may state that a flight attendant should perform alcohol detection before and after an operation time period and a pilot, on the other hand, should perform alcohol detection only before an operation time period. If operating hours are to be long as in the case with an international flight or the like, the rule may state that alcohol detection should be performed within a specified time period during the operation time period.

The authentication server 16 comprises a data sending and receiving unit 80, a storage 82, an ID determination unit 84, a face authentication unit 86, a transport-related-work go/no-go determination unit 88, and a performance status determination unit 90.

The data sending and receiving unit 80 receives an alcohol measurement value, a device ID, the manufacturing ID, the total measurement number, camera image information, and the like sent from the tablet terminal 14, and sends an authentication result, a transport-related work go/no-go result, and the like to the tablet terminal 14.

The storage 82 holds a person-to-be-measured list and a device list. On the person-to-be-measured list, various pieces of registration information including the name, affiliation, and the like of a person to be measured are registered along with image information indicating the face of the person to be measured (hereinafter referred to as "registered face image information") for each registered person. On the device list, the manufacturing IDs of the alcohol detection devices 12 used in the alcohol detection system 10 are registered. Which alcohol detection device 12 a person to be measured is to use may be determined in advance, and in that case the manufacturing ID of the alcohol detection device 12 to be used by the person to be measured is also registered on the person-to-be-measured list.

The storage 82 also holds for each person to be measured an alcohol measurement value, camera image information, a transport-related work go/no-go result, and the like sent from the tablet terminal 14, and holds for each alcohol detection device 12 the total measurement number sent from the tablet terminal 14. The total measurement number for each alcohol detection device 12 stored in the storage 82 is referred to as the server-stored total number.

The ID determination unit 84 extracts an indication ID from camera image information. The ID determination unit 84 then determines whether a person to be measured is performing alcohol detection using the alcohol detection device 12 an image of which has been shot by the camera 32 or not based on the extracted indication ID and a device ID sent from the alcohol detection device 12. The ID determination unit 84 of the embodiment determines that the person to be measured is performing alcohol detection using the alcohol detection device 12 an image of which has been shot by the camera 32 if the indication ID and the device ID are identical. The ID determination unit 84 also determines whether the manufacturing ID sent from the tablet terminal 14 is included on the device list stored in the storage 82 or not.

The face authentication unit 86 of the embodiment performs face authentication on a person to be measured based on face image information that is indicated by camera image information and indicates the face of the person to be measured. The face authentication unit 86 of the embodiment performs the face authentication on a person to be measured with face image information that is indicated by camera image information and indicates the face of the person to be measured and registered face image information that is registered in advance on the person-to-be-measured list. The face authentication unit 86 also has a function to extract the face of a person to be measured from camera image information to acquire face image information.

The transport-related-work go/no-go determination unit 88 determines the state of alcohol ingestion of a person to be measured based on an alcohol measurement value and a predetermined reference value and, based on the determination, determines whether the person to be measured can be allowed to board for service or not. That is, if the alcohol measurement value exceeds the reference value, the relevant person to be measured is prohibited from boarding for service.

The performance status determination unit 90 determines whether alcohol detection is being properly performed or not based on measurement performance information sent from the alcohol detection device 12. The performance status determination unit 90 of the embodiment determines whether alcohol detection is being properly performed by the alcohol detection device 12 or not based on, for example, the total measurement number sent from the alcohol detection device 12 and the server-stored total number stored in the storage 82. The performance status determination unit 90 of the embodiment determines the detection to be proper if the total measurement number and the server-stored total number are identical.

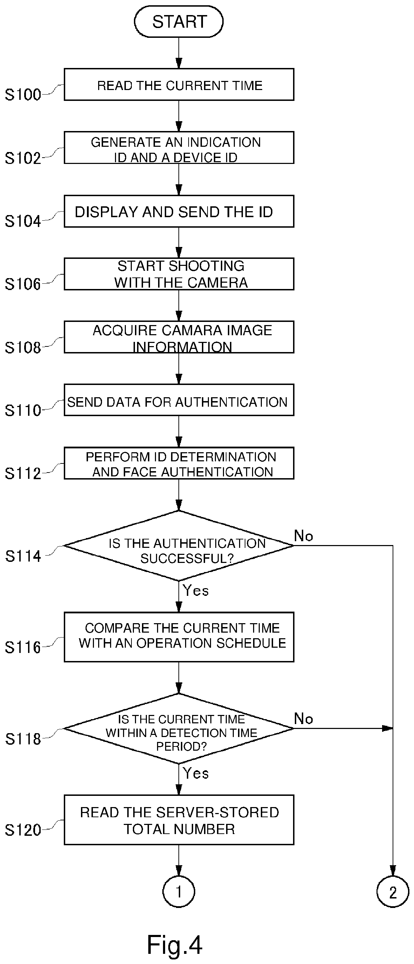

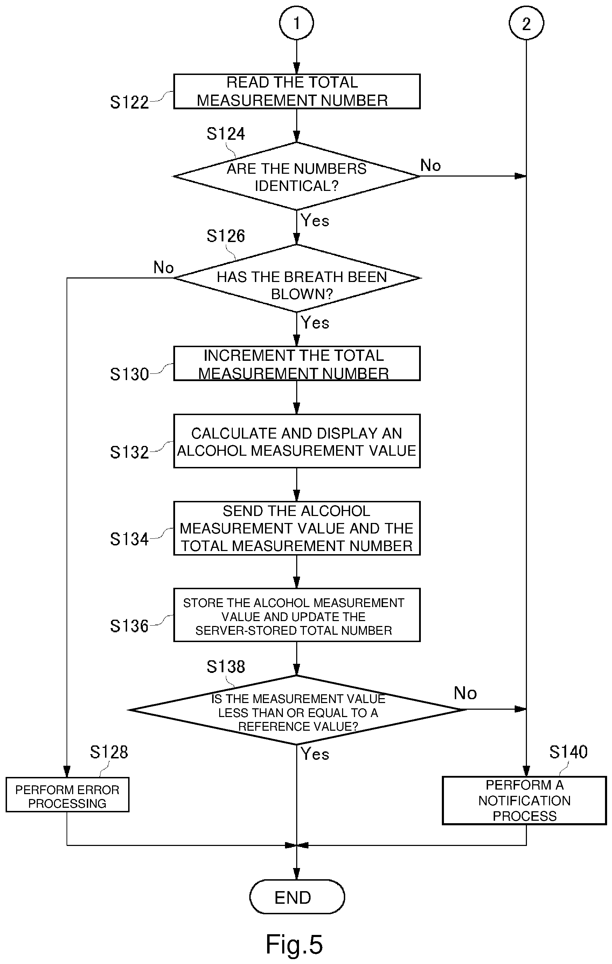

Figures 4 and 5 are flowcharts showing a flow of an alcohol detection process (an alcohol detection program) that is executed in the alcohol detection system 10 of the embodiment. The alcohol detection process is started when the alcohol detection app is started on the tablet terminal 14 and recognizes a connection between the alcohol detection device 12 that is powered on and the tablet terminal 14. The alcohol detection program may be provided to the alcohol detection system 10 by the alcohol detection system 10 (the alcohol detection device 12, the tablet terminal 14, and the authentication server 16) downloading it from a communication network, or may be provided to the alcohol detection system 10 via a non-transitory recording medium.

First, in a step S100, the operation time period determination unit 70 of the tablet terminal 14 reads the current time T1.

In the next step S102, the ID generator 44 of the alcohol detection device 12 generates an indication ID and a device ID.

In the next step S104, the image display controller 46 of the alcohol detection device 12 causes the display 22 to display the device ID, and the data sending and receiving unit 52A sends the device ID to the tablet terminal 14. The alcohol detection device 12 sends the manufacturing ID to the tablet terminal 14 along with the device ID. The tablet terminal 14 therefore causes the storage 66 to hold the device ID and camera image information sent from the alcohol detection device 12 in association with the received manufacturing ID.

In the next step S106, an operation performed on the measurement start button of the alcohol detection device 12 triggers the camera controller 62 of the tablet terminal 14 to shoot an image with the camera 32. The image shot by the camera 32 is displayed on the touch-panel display 30 of the tablet terminal 14 during the shooting, and the person to be measured adjusts the position of the person themself and the alcohol detection device 12 so that the face of the person themself and the display 22 of the alcohol detection device 12 are included in the angle of view 36 of the camera 32.

In the next step S108, the camera controller 62 acquires the image shot by the camera 32 as camera image information when the camera 32 starts the shooting. The acquisition of camera image information just mentioned means storing the image shot by the camera 32 in a non-volatile storage device comprised in the tablet terminal 14.

Though in the embodiment an operation performed on the measurement start button triggers the start of the shooting with the camera 32 and the acquisition of camera image information as described above, the start trigger for the acquisition of camera image information is not limited to this. For example, the start of the shooting with the camera 32 and the recognition of the face of a person being included in the shot image may trigger the start of the acquisition of camera image information. The start of the shooting with the camera 32 and the recognition of an indication ID being included in the shot image may also trigger the start of the acquisition of camera image information.

In the next step S110, the data sending and receiving unit 68 of the tablet terminal 14 sends the device ID and the manufacturing ID received from the alcohol detection device 12 and the camara image information shot by the camera 32 to the authentication server 16 as data for authentication.

In the next step S112, the ID determination unit 84 of the authentication server 16 performs ID determinations, and the face authentication unit 86 performs face authentication. Whether the indication ID indicated by the camera image information and the device ID sent from the tablet terminal 14 are identical or not is determined as an ID determination. Whether the manufacturing ID sent from the tablet terminal 14 is included on the device list or not is also determined as an ID determination.

The face authentication of the embodiment is performed on a person to be measured with face image information that indicates the face of the person to be measured and registered face image information that is registered on the person-to-be-measured list. The embodiment thus allows the face authentication to be performed along with the authentication using the indication ID and the device ID, and therefore allows a more reliable determination of whether the measurement is performed by the person to be measured themself or not. The face authentication may also be performed not by comparing face image information with registered face image information, but by acquiring a plurality of pieces of camera image information and determining whether a plurality of pieces of face image information included in the pieces of camera image information belong to an identical person or not.

In the next step S114, whether the ID determinations and the face authentication are successful or not is determined. The process proceeds to a step S116 if the determination is affirmative, and proceeds to a step S140 If the determination is negative. The result of the ID determinations and the face authentication (the authentication result) is sent to the tablet terminal 14. When the authentication result is unsuccessful, the tablet terminal 14 displays an image indicating the failure on the touch-panel display 30, and thereby allows the person to be measured to recognize the authentication to be unsuccessful.

In the step S116, the operation time period determination unit 70 of the tablet terminal 14 compares the current time T1 read by the operation time period determination unit 70 in the step S100 with an operation schedule.

In the next step S118, the process proceeds to a step S120 if the current time T1 is within an alcohol detection time period, and proceeds to the step S140 If a negative determination is made.

In the step S120, the performance status determination unit 90 reads the server-stored total number stored in the storage 82 of the authentication server 16. The server-stored total number to be read is of the alcohol detection device 12 connected to the tablet terminal 14, and is associated with the manufacturing ID.

In the next step S122, the performance status determination unit 90 reads the total measurement number via the tablet terminal 14 from the alcohol detection device 12.

In the next step S124, the performance status determination unit 90 determines whether the total measurement number and the server-stored total number are identical or not. The process proceeds to a step S126 if the determination is affirmative, and proceeds to the step S140 If the determination is negative. If the determination is affirmative, an authentication result indicating that the numbers are identical is sent to the tablet terminal 14. Upon the tablet terminal 14 receiving the authentication result, the alcohol detection app causes the touch-panel display 30 to display an image urging the person to be measured to start blowing the breath into the alcohol detection device 12 (hereinafter referred to as the "detection start image").

In the next step S126, the alcohol concentration measuring unit 42 of the alcohol detection device 12 determines whether the person to be measured has blown the breath or not. The process proceeds to a step S130 if the determination is affirmative, and proceeds to a step S128 If the determination is negative. A negative determination is made when, for example, the measurement start button of the alcohol detection device 12 is not operated and the breath is not blown within a specified period (e.g., within three minutes) after the detection start image has been displayed on the tablet terminal 14.

In the step S128, redetection information indicating that alcohol detection is performed again from the step S100 is sent from the alcohol detection device 12 to the tablet terminal 14 and the authentication server 16 as error processing. Based on the redetection information, the alcohol detection app causes the touch-panel display 30 to display an image urging to blow the breath.

In the step S130, the measurement performance information generator 48 of the alcohol detection device 12 increments the total measurement number by one, following the alcohol concentration measuring unit 42 having detected the blowing of the breath.

In the next step S132, the alcohol concentration measuring unit 42 calculates an alcohol measurement value from the breath alcohol concentration, and the image display controller 46 of the alcohol detection device 12 causes the display 22 to display the alcohol measurement value.

In the next step S134, the data sending and receiving unit 52 of the alcohol detection device 12 sends the alcohol measurement value and the total measurement number to the tablet terminal 14. The tablet terminal 14 sends the received alcohol measurement value and total measurement number to the authentication server 16.

In the next step S136, the authentication server 16 causes the storage 82 to hold the received alcohol measurement value for each person to be measured. The authentication server 16 also replaces the server-stored total number stored in the storage 82 with the value of the received total measurement number.

The determination process performed on the basis of the server-stored total number and the total measurement number in the step S124 is described here in detail. First, some time (e.g., about a few tens of seconds) is required for the alcohol detection device 12 to perform the measurement starting from blowing of the breath and ending in determination of the alcohol concentration. A person to be measured, who has sensed that the alcohol concentration in the person's breath is high after the person blew the breath, may therefore disconnect the connection (communication) between the alcohol detection device 12 and the tablet terminal 14 while the alcohol measurement value is being calculated. A person to be measured may also turn off the alcohol detection device 12 while an alcohol measurement value is being calculated. Acts like these indicate that the person to be measured may have ingested alcohol that exceeded the reference value before or after boarding an aircraft for service, and are considered fraudulent acts. A person to be measured, who is aware that the person themself is under the influence of alcohol, may perform alcohol detection and check if the alcohol measurement value does not exceed the reference value without connecting the alcohol detection device 12 to the tablet terminal 14. Such an act is also considered a fraudulent act in the embodiment.

Secondly, since in the alcohol detection process of the embodiment the total measurement number is sent to the authentication server 16 after calculation of an alcohol measurement value as in the step S134, the server-stored total number stored in the authentication server 16 is not updated if the above-described fraudulent acts are carried out. Accordingly, the total measurement number not being identical with the server-stored total number in the step S124 indicates a probability of fraudulent acts like those described above.

In this way, the alcohol detection process of the embodiment compares the server-stored total number with the total measurement number, and can thereby determine whether a proper measurement has been performed by the alcohol detection device 12 without any fraudulent act such as disconnection of the alcohol detection device 12 from the tablet terminal 14 or not.

In the next step S138, the transport-related-work go/no-go determination unit 88 of the authentication server 16 determines the state of alcohol ingestion of the person to be measured based on the alcohol measurement value and the reference value, and the alcohol detection process ends if the alcohol measurement value is less than or equal to the reference value, and proceeds to the step S140 if the determination is negative. When the affirmative determination is made, the fact that the alcohol detection shows that the person to be measured is free of irregularities and can board an aircraft for service is displayed on the touch-panel display 30 of the tablet terminal 14. When the negative determination is made, on the other hand, the fact that the state of alcohol ingestion exceeds the reference value and the person to be measured cannot board an aircraft for service is displayed on the touch-panel display 30.

In the step S140, the authentication server 16 performs a notification process. If a negative determination is made in the step S114, S118, or S124, the notification process sends information indicating that boarding for service is prohibited due to failure in authentication to a management server or a management terminal along with the reason for the failure in authentication. If a negative determination is made in the step S138, the notification process sends information indicating that boarding for service is prohibited due to the state of alcohol ingestion exceeding the reference value to a management server or a management terminal.

### (Second embodiment)

A second embodiment is described below. The alcohol detection device 12 of this embodiment is provided with an accelerometer, whose detection result is used to determine fraudulent acts. Fraudulent acts are also determined by performing face authentication on a person to be measured more than once in the embodiment.

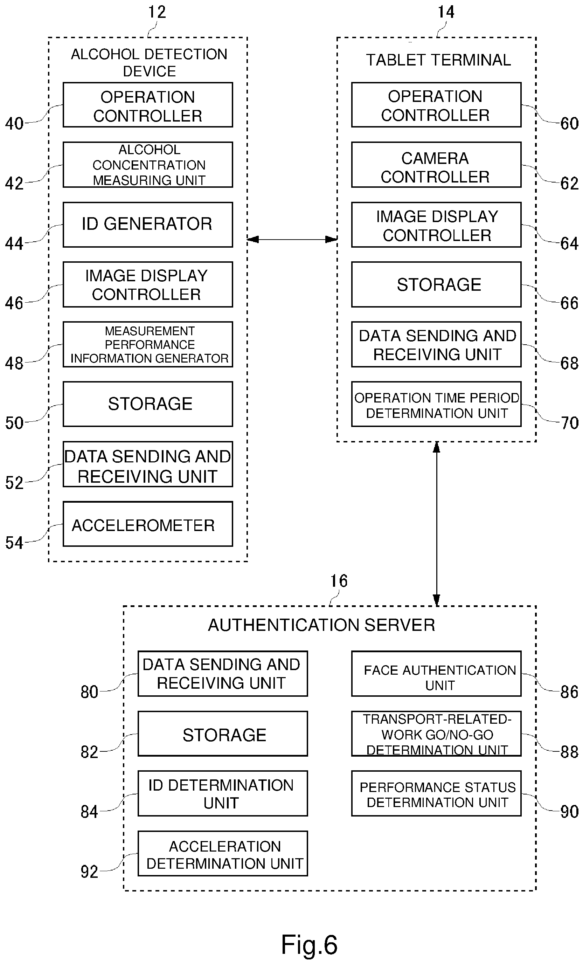

Figure 6 is a function block diagram of the alcohol detection system 10 of the embodiment. The same components in Figure 6 as in Figure 2 are designated by the same symbols, and their descriptions are omitted.

An accelerometer 54 comprised in the alcohol detection device 12 of the embodiment detects the acceleration produced in the alcohol detection device 12 during alcohol detection performed by the alcohol detection device 12. The acceleration detected by the accelerometer 54 is outputted as acceleration information.

The authentication server 16 of the embodiment is provided with an acceleration determination unit 92. The acceleration determination unit 92 determines that a person to be measured is not using the alcohol detection device 12 an image of which has been shot by the camera 32 to perform alcohol detection if the accelerometer 54 detects an acceleration greater than or equal to a specified value while the alcohol detection device 12 is performing alcohol detection. Note that the specified value is a value that allows determination of an instantaneous movement of the alcohol detection device 12, and is arbitrarily set.

The accelerometer 54 detecting an acceleration greater than or equal to a specified value while the alcohol detection device 12 is performing alcohol detection, which is mentioned above, means that the alcohol detection device 12 is moved instantaneously, and the alcohol detection device 12 may be handed from the person to be measured to another person. Such an act is considered performed with a fraudulent intention. The determination with the acceleration determination unit 92 therefore allows determination of whether a fraudulent act has been committed or not.

The camera 32 of the embodiment shoots an image of the face of a person to be measured more than once, and the face authentication unit 86 performs face authentication based on a plurality of pieces of face image information whose images have been shot continuously. In this way, the alcohol detection system 10 of the embodiment allows the face authentication to be performed by using a plurality of pieces of face image information, and therefore allows a more reliable determination of whether the measurement is performed by the person to be measured themself or not.

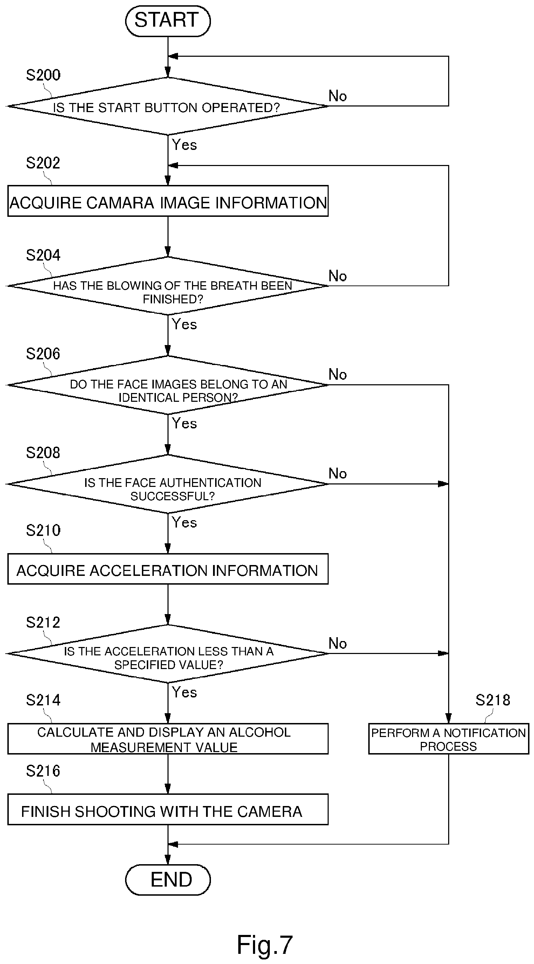

Figure 7 is a flowchart showing a flow of an alcohol detection process (an alcohol detection program) that is executed in the alcohol detection system 10 of the embodiment. Note that Figure 7 shows a process of fraudulent act determination using acceleration and face authentication using a plurality of shot face images, and the other authentication processes, the incrementing of the total measurement number, and the like described in the first embodiment are omitted but are executed in the same manner as in the first embodiment. The alcohol detection device 12 starts detecting acceleration with the accelerometer 54 when, for example, the power is turned on.

First, in a step S200, the operation controller 40 determines whether the measurement start button of the alcohol detection device 12 is operated or not, and the process proceeds to a step S202 if the determination is affirmative.

In the next step S202, the camera controller 62 of the tablet terminal 14 starts shooting with the camera 32 and acquires camera image information. The camera image information is sent to the authentication server 16 via the data sending and receiving unit 68. The authentication server 16 causes the storage 82 to hold the received camera image information. Note that the acquisition of camera image information with the camera 32 is performed at specified time intervals (e.g., at intervals of 500 msec). The specified time intervals only have to be, for example, those that allow determination of swapping of the alcohol detection device 12 during the shooting.

In the next step S204, the alcohol concentration measuring unit 42 determines whether the person to be measured has finished blowing the breath or not, and the process proceeds to a step S206 if the determination is affirmative. If the determination is negative, on the other hand, the process returns to the step S204 and the acquisition of camera image information is continuously repeated until the blowing of the breath finishes.

In the step S206, the face authentication unit 86 extracts pieces of face image information from a plurality of pieces of camera image information, and determines whether a plurality of face images indicated by the pieces of face image information belong to an identical person or not. The process proceeds to a step S208 if the determination is affirmative, and proceeds to a step S218 if the determination is negative. At least only two pieces of camera image information are required for use in the extraction of face image information.

In the step S208, the face authentication unit 86 performs face authentication, and the process proceeds to a step S210 if the face authentication is successful, and proceeds to the step S218 if the face authentication is unsuccessful. At least only one piece of face image information is required for use in the face authentication, and two or more pieces of face image information may be used.

In the step S210, the acceleration determination unit 92 acquires acceleration information outputted from the accelerometer 54 from the alcohol detection device 12 via the tablet terminal 14. Note that the acceleration information is acquired for a period corresponding to a period during which the person to be measured blew the breath into the alcohol detection device 12.

In the next step S212, the acceleration determination unit 92 determines whether the value of the acquired acceleration information (acceleration) is less than a specified value or not, and the process proceeds to a step S214 if the determination is affirmative, and proceeds to the step S218 if the determination is negative.

In the step S214, the alcohol concentration measuring unit 42 calculates an alcohol measurement value from the breath alcohol concentration, and the image display controller 46 of the alcohol detection device 12 causes the display 22 to display the alcohol measurement value.

In the next step S216, the camera controller 62 finishes shooting with the camera 32.

In the step S218, the authentication server 16 performs a notification process. The notification process sends information indicating that boarding for service is prohibited due to failure in authentication to a management server or a management terminal along with the reason for the failure in authentication. The notification process sends information indicating that boarding for service is prohibited due to a fraudulent act to a management server or a management terminal.

While a description has been made for the embodiment on modes in which the camera 32 shoots a plurality of still images, the embodiment is not limited to this. There may be a mode in which the camera 32 shoots a moving image until the blowing of the breath is finished, pieces of face image information of a person to be measured in the moving image at different times are extracted, and whether the extracted pieces of face image information belong to an identical person or not is determined.

While a description has been made for the embodiment on modes in which an operation performed on the measurement start button triggers the start of the acquisition of camera image information with the camera 32, the embodiment is not limited to this. For example, the start of the acquisition of camera image information may be triggered by the start of the alcohol detection app, an operation to turn on the power of the alcohol detection device 12 after the start of the alcohol detection app, detection of acceleration with the accelerometer 54 of the alcohol detection device 12, sensing performed by another sensor (e.g., a gyroscope sensor) of the alcohol detection device 12, or activation of a reed switch or the like comprised in the alcohol detection device 12.

### (Third embodiment)

A third embodiment is described below. A moving image of a person to be measured is used for determination of a fraudulent act in this embodiment.

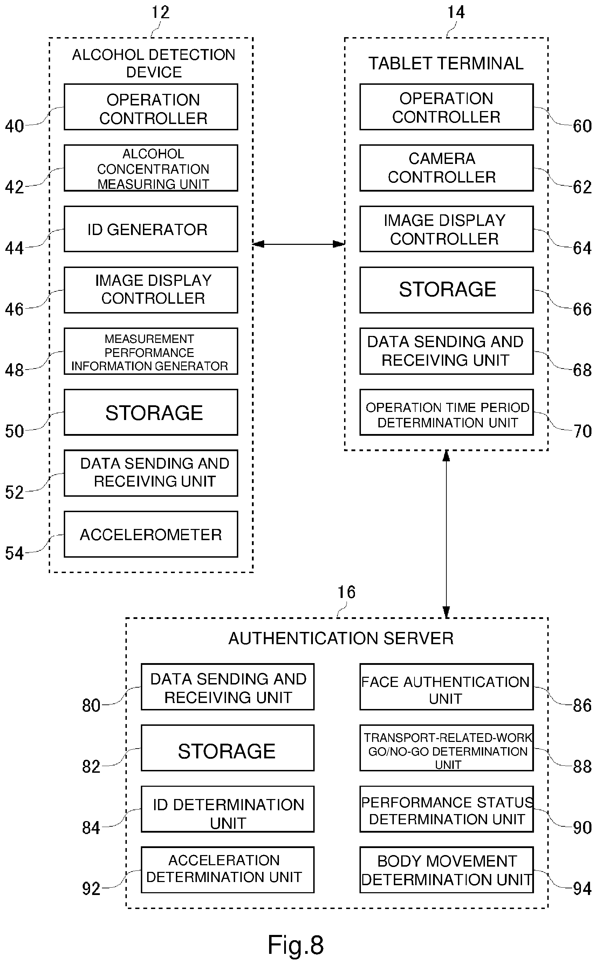

Figure 8 is a function block diagram of the alcohol detection system 10 of the embodiment. The same components in Figure 8 as in Figures 2 and 6 are designated by the same symbols, and their descriptions are omitted.

Now, there may be an act in which, as a fraudulent alcohol-detection act, another person than a person to be measured causes the camera 32 to shoot a face image or the like of the person to be measured and pretends to be the person to be measured to perform alcohol detection.

The authentication server 16 of the embodiment is therefore provided with a body movement determination unit 94. The body movement determination unit 94 determines whether there is a movement (hereinafter referred to as a "body movement") in a person to be measured or not based on camera image information acquired by the camera 32 of the tablet terminal 14. This allows determination of whether the person to be measured who is being shot by the camera 32 is a living body or not, and therefore allows a more reliable determination of whether the measurement is performed by the person to be measured themself or not. An example of the camera image information used for the determination performed by the body movement determination unit 94 is moving image information that indicates a moving image. That is, the camera 32 of the embodiment acquires, for example, moving image information. Movements of a person to be measured to be determined as body movements include, for example, a blink of an eye, the movement of the mouth, and the sway of the head or body. The determination of whether there is a body movement in a person to be measured or not performed by the body movement determination unit 94 is also called body movement determination.

The body movement determination unit 94 also determines whether alcohol detection is properly performed by the alcohol detection device 12 or not based on time during which the breath is being blown (hereinafter referred to as a "breath blowing time") and time during which there is a body movement in a person to be measured (hereinafter referred to as a "body movement detection time"). That is to say, for example, determination of whether the difference between a breath blowing time and a body movement detection time falls within a specified range or not allows determination of whether alcohol detection is properly performed by the alcohol detection device 12 or not, since the mouth or body of a person to be measured moves when the person to be measured blows the breath into the alcohol detection device 12.

In addition, the body movement determination unit 94 may also determine whether alcohol detection is properly performed by the alcohol detection device 12 or not based on acceleration information detected by the accelerometer 54 of the alcohol detection device 12 and moving image information. That is, when a person to be measured blows the breath into the alcohol detection device 12, alcohol detection device 12 vibrates. The accelerometer 54 therefore detects acceleration when there is a body movement, and whether alcohol detection is properly performed or not can be determined based on these pieces of information. The body movement determination unit 94 determines whether alcohol detection is properly performed by the alcohol detection device 12 or not based on, for example, time during which the accelerometer 54 is detecting acceleration (hereinafter referred to as an "acceleration detection time") and a body movement detection time.

Note that a body movement detection time, a breath blowing time, and an acceleration detection time conceptually have their own lengths. That is, for example, if the above-mentioned specified range is set to be eight seconds and a body movement detection time of 13 seconds and a breath blowing time of 15 seconds are obtained, the body movement determination unit 94 determines that the alcohol detection is properly performed. If a body movement detection time of one second and a breath blowing time of 15 seconds are obtained, on the other hand, the body movement determination unit 94 determines that the alcohol detection is not properly performed.

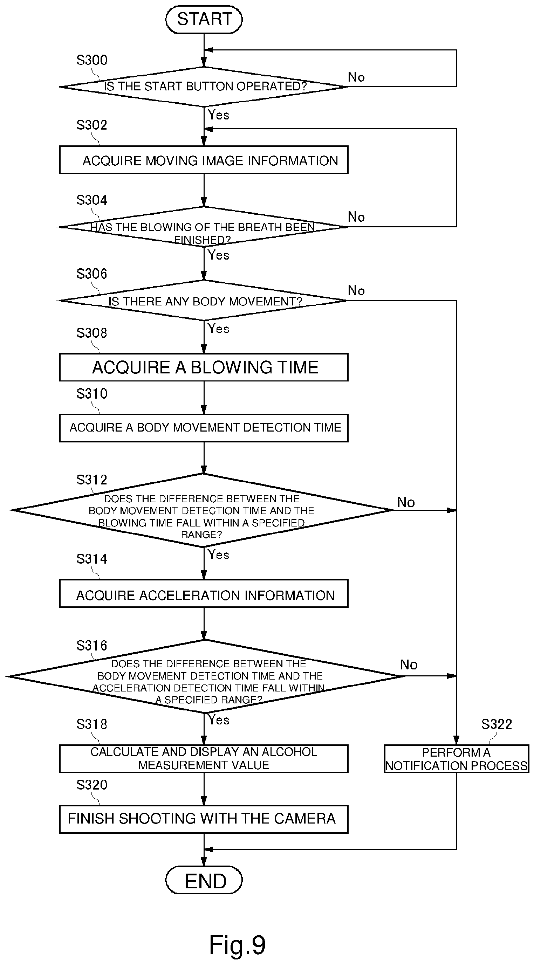

Figure 9 is a flowchart showing a flow of an alcohol detection process (an alcohol detection program) that is executed in the alcohol detection system 10 of the embodiment. Note that Figure 9 shows a process of fraudulent act determination using acceleration and face authentication for a body movement, and the other authentication processes, the incrementing of the total measurement number, and the like described in the first embodiment are omitted but are executed in the same manner as in the first embodiment. The alcohol detection device 12 starts detecting acceleration with the accelerometer 54 when, for example, the power is turned on.

First, in a step S300, the operation controller 40 determines whether the measurement start button of the alcohol detection device 12 is operated or not, and the process proceeds to a step S302 if the determination is affirmative.

In the next step S302, the camera controller 62 of the tablet terminal 14 starts shooting a moving image with the camera 32 and acquires moving image information. The moving image information is sent to the authentication server 16 via the data sending and receiving unit 68. The authentication server 16 causes the storage 82 to hold the received moving image information.

In the next step S304, the alcohol concentration measuring unit 42 determines whether the person to be measured has finished blowing the breath or not, and the process proceeds to a step S306 if the determination is affirmative. If the determination is negative, on the other hand, the process returns to the step S302 and the acquisition of moving image information is repeated until the blowing of the breath finishes.

In the step S306, the body movement determination unit 94 determines whether there is a movement in the person to be measured indicated by the moving image information or not, and the process proceeds to a step S308 if the determination is affirmative, and proceeds to a step S322 if the determination is negative.

In the step S308, the body movement determination unit 94 acquires a blowing time from the alcohol detection device 12 via the tablet terminal 14.

In a step S310, the body movement determination unit 94 acquires a body movement detection time from the moving image information.

In the next step S312, the body movement determination unit 94 determines whether the difference between the body movement detection time and the blowing time falls within a specified range or not, and the process proceeds to a step S314 if the determination is affirmative, and proceeds to the step S322 if the determination is negative.

In the next step S314, the acceleration determination unit 92 acquires acceleration information outputted from the accelerometer 54 via the tablet terminal 14. The acceleration determination unit 92 extracts an acceleration detection time based on the acquired acceleration information.

In a step S316, the body movement determination unit 94 determines whether the difference between the body movement detection time and the acceleration detection time falls within a specified range or not, and the process proceeds to a step S318 if the determination is affirmative, and proceeds to the step S322 if the determination is negative.

In the step S318, the alcohol concentration measuring unit 42 calculates an alcohol measurement value from the breath alcohol concentration, and the image display controller 46 of the alcohol detection device 12 causes the display 22 to display the alcohol measurement value.

In the next step S320, the camera controller 62 finishes shooting with the camera 32.

In the step S322, the authentication server 16 performs a notification process. The notification process sends information indicating that boarding for service is prohibited due to a fraudulent act to a management server or a management terminal.

While the body movement determination unit 94 of the embodiment determines whether there is a body movement in a person to be measured or not based on time during which there is a body movement in the person to be measured (a body movement detection time), the embodiment is not limited to this. For example, whether there is a body movement in a person to be measured or not may be determined based on the amount of movement of the person to be measured. The amount of movement of a person to be measured is, for example, the amount of movement of the person's eyeball or the amount of movement of the person's head.

While the disclosure has been described with reference to the above embodiments, the technical scope of the disclosure is not limited to the scope provided by the embodiments. Various modifications or improvements can be made to the embodiments without departing from the gist of the disclosure, and those added with the modifications or improvements are also included in the technical scope of the disclosure. The components and flowchart of each of the above embodiments may be embodied in combination with one another as appropriate.

For example, while a description has been made for the above embodiments on modes in which the breath alcohol concentration of a person to be measured is detected as biological information of the person to be measured, the embodiment is not limited to this, and other biological information may be used, for example, as long as the biological information can be detected from the breath of a person to be measured. Furthermore, the biological information is not limited to those that are detected from the breath of a person to be measured, and may be biological information that can be detected from a subject's sample such as blood, saliva, and part of a tissue.

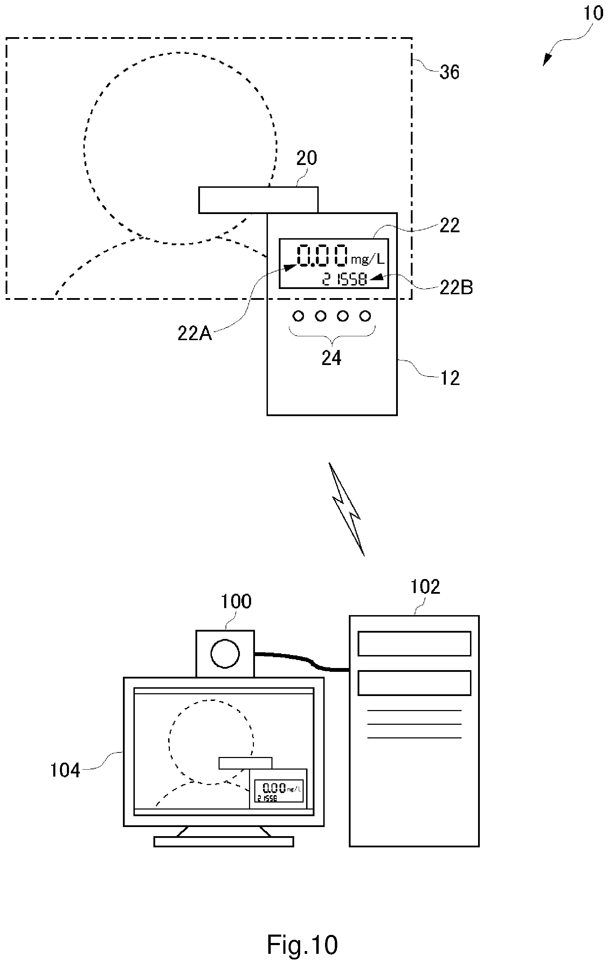

While a description has been made for the above embodiments on modes in which the alcohol detection system 10 comprises the alcohol detection device 12, the tablet terminal 14, and the authentication server 16, the embodiment is not limited to this. For example, the alcohol detection system 10 may comprise the alcohol detection device 12, a camera device 100, and an information-processing device 102 as shown in Figure 10.

The information-processing device 102 is provided with a display 104, and is connected with the camera device 100. That is, the information-processing device 102 receives camera image information acquired by the camera device 100 shooting an image of an indication ID displayed on the display 22 of the alcohol detection device 12 and a device ID sent from the alcohol detection device 12 in the example in Figure 10. The information-processing device 102 then determines whether a person to be measured is performing alcohol detection using the alcohol detection device 12 an image of which has been shot by the camera 32 or not based on the indication ID indicated by the camera image information and the device ID sent from the alcohol detection device 12. In the example in Figure 10, the alcohol detection device 12 and the information-processing device 102 send and receive data between each other via wireless communication.

While a description has been made for the above embodiments on modes in which an indication ID is a random number, the embodiment is not limited to this. An indication ID may be, for example, an alcohol measurement value itself indicating a measurement result obtained by the alcohol detection device 12. Note that this configuration allows determination of whether the alcohol detection device 12 an image of which has been shot is the very device used for performing alcohol detection on a person to be measured or not with simpler components, since an alcohol measurement value is generated for each measurement. In this mode, the display 22 of the alcohol detection device 12 is not provided with the ID display area 22B shown in Figure 1.

A plurality of indication IDs, for example, may be set and be stored in the storage 50 of the alcohol detection device 12 in advance, and one of those may be chosen as an indication ID each time alcohol detection is performed.

There may be a mode in which an indication ID is converted to a one-dimensional code (a bar code) or a two-dimensional code (a QR code (registered trademark)) corresponding to a device ID and is displayed. In this mode, the above code is shot by the camera 32 and is then converted to a numeric or the like to be used for authentication. That is, an indication ID and a device ID in the embodiments have only to correspond to each other, and an indication ID and a device ID may be determined to be identical if there is the correspondence. The correspondence is not limited to the above, and there is an example in which a device ID is set to be of a fixed value and an indication ID is set to be of a value obtained by adding or subtracting a predetermined numerical value to or from the value of the device ID. A device ID may also be set to be identical with the manufacturing ID.

An indication ID may include information on the authenticated face of a person to be measured (hereinafter referred to as "face authentication information"). In this mode, face authentication is performed on a person to be measured before the alcohol detection device 12 displays an indication ID, and the result of the face authentication is sent to the alcohol detection device 12. The alcohol detection device 12 then generates an indication ID, including face authentication information in the indication ID. Face authentication information is, for example, a time at which face authentication was performed, information previously associated with registered face image information, or the like. When an indication ID includes face authentication information, the indication ID is displayed, for example, as a two-dimensional code in the ID display area 22B.

The alcohol detection device 12 may be provided with an LED display formed of a plurality of LEDs (Light Emitting Diodes), and an indication ID may be expressed by a lighting pattern of the LEDs.

While a description has been made for the above embodiments on modes in which measurement performance information is the total number of alcohol detection performed by the alcohol detection device 12, the embodiment is not limited to this. For example, measurement performance information may be a flag indicating a success or a failure in alcohol detection (hereinafter referred to as a "detection flag"), or may be data items that are sent from the alcohol detection device 12 as alcohol detection is performed.

A detection flag is generated by the tablet terminal 14. Specifically, a detection flag indicating a failure is generated if the alcohol detection device 12 is disconnected from the tablet terminal 14 before the alcohol detection device 12 finishes alcohol detection. Note that the alcohol detection device 12 finishing alcohol detection means the alcohol detection device 12 completing sending an alcohol measurement value to the tablet terminal 14. That is, a detection flag indicating a success is set if the alcohol detection device 12 completes sending an alcohol measurement value to the tablet terminal 14. The performance status determination unit 90 then determines that the alcohol detection is properly performed if the detection flag indicates a success, but determines that the alcohol detection is not properly performed if the detection flag indicates a failure.

The data items are those sent from the alcohol detection device 12 while alcohol detection is being performed. The data items include, for example, a time when the alcohol detection device 12 started alcohol detection, an alcohol measurement value, and a time when alcohol detection was finished, the types (number) of which are (is) specified in advance. Those data items are sequentially sent to the tablet terminal 14 between the start and finish of alcohol detection. That is to say, the number of data items received by the tablet terminal 14 is less than the specified number if the alcohol detection device 12 is disconnected from the tablet terminal 14 before the alcohol detection device 12 finishes alcohol detection. The performance status determination unit 90 therefore determines that the alcohol detection is properly performed if the number of data items received by the tablet terminal 14 is equal to the specified number, but determines that the alcohol detection is not properly performed if the number of data items received by the tablet terminal 14 is less than the specified number.

While a description has been made for the above embodiments on modes in which a person to be measured is a crew member of an aircraft, the embodiment is not limited to this. A person to be measured may be a driver of a business vehicle such as a bus, a taxi, a truck, and a motorcycle, or may be a crew member of a ship or a railroad train. A person to be measured is not limited to a crew member of an aircraft or a driver of a business vehicle, and may also be a worker working at or a manager managing a hospital, a factory, a store, an office, or the like.

While a description has been made for the above embodiments on modes in which one indication ID is displayed on the alcohol detection device 12 for one time of alcohol concentration measurement, the embodiment is not limited to this. An indication ID may be generated and displayed, for example, two or more times for one time of alcohol concentration measurement. For example, an indication ID may be generated and displayed when an alcohol concentration measurement is started, and when a result of the alcohol concentration measurement is calculated, an indication ID including the measurement result may be generated and displayed. An indication ID may also be displayed, for example, always on a body of the alcohol detection device 12 other than the display 22, or an updated indication ID may be newly generated at specified time intervals (e.g., one-minute intervals) and be displayed in the ID display area 22B of the alcohol detection device 12. Incidentally, the ID generator 44 may generate a device ID, for example, concurrently with the generation of an indication ID.

### DESCRIPTION OF THE SYMBOLS

10: Alcohol detection system (Biological information measuring system)
12: Alcohol detection device (Biological information measuring device)
16: Authentication server (Information-processing device)
22: Display (Display means)
32: Camera (Image-shooting device)
52: Data sending and receiving unit (Sending means)
54: Accelerometer
80: Data sending and receiving unit (Receiving means)
86: Face authentication unit (Face authentication means)
88: Transport-related-work go/no-go determination unit (Determination means)
90: Performance status determination unit (Performance status determination means)
94: Body movement determination unit (Body movement determination means)

## Claims

1. A biological information measuring system comprising:
a biological information measuring device for measuring biological information of a person to be measured;
an image-shooting device; and
an information-processing device, wherein the biological information measuring device comprises:
display means for displaying indication identification information; and
sending means for sending device identification information, and wherein the information-processing device comprises:
receiving means for receiving
image information acquired by the image-shooting device shooting an image of the indication identification information displayed on the display means and
the device identification information sent from the sending means; and
determination means for determining whether the person to be measured is measuring the biological information using the biological information measuring device an image of which has been shot by the image-shooting device or not based on the indication identification information indicated by the image information and the device identification information sent from the sending means.

2. The biological information measuring system according to claim 1, wherein the biological information of the person to be measured is breath alcohol concentration of the person to be measured.

3. The biological information measuring system according to claim 1 or 2, wherein the indication identification information is randomly generated by the biological information measuring device each time the biological information is measured.

4. The biological information measuring system according to claim 1 or 2, wherein the indication identification information is a value indicating a measurement result obtained by the biological information measuring device.

5. The biological information measuring system according to any one of claims 1 to 4, wherein the image-shooting device shoots an image of a face of the person to be measured who is performing measurement with the biological information measuring device as well as the image of the indication identification information displayed on the display means, and wherein the information-processing device comprises face authentication means for performing face authentication on the person to be measured based on face image information that is indicated by the image information and indicates the face of the person to be measured.

6. The biological information measuring system according to claim 5, wherein the face authentication means performs the face authentication based on a plurality of pieces of face image information, images of which have been shot continuously.

7. The biological information measuring system according to claim 5 or 6, wherein the face authentication means performs face authentication on the person to be measured with the face image information and registered face image information that is registered in advance and indicates the face of the person to be measured.

8. The biological information measuring system according to any one of claims 1 to 7, comprising body movement determination means for determining whether the person to be measured is moving or not based on the image information.

9. The biological information measuring system according to any one of claims 1 to 8,
wherein each time the biological information is measured, the biological information measuring device sends measurement performance information that is information on how the measurement of the biological information is performed to the information-processing device, and
wherein the information-processing device comprises performance status determination means for determining whether the biological information measuring device is properly measuring the biological information or not based on the measurement performance information.

10. The biological information measuring system according to any one of claims 1 to 9, wherein the biological information of the person to be measured is measured during a predetermined time period.

11. The biological information measuring system according to claim 10,
wherein the person to be measured is a crew member of an aircraft, and
wherein the predetermined time period is at least one of specified time periods before and after an operation time period of the aircraft that the crew member boards for service.

12. The biological information measuring system according to any one of claims 1 to 11,
wherein the biological information measuring device comprises an accelerometer, and
wherein if the accelerometer detects an acceleration greater than or equal to a specified value while the biological information measuring device is performing the measurement, the determination means determines that the person to be measured is not measuring the biological information using the biological information measuring device an image of which has been shot by the image-shooting device.

13. A fraudulent-measurement determination method being an authentication method for a biological information measuring system comprising: a biological information measuring device for measuring biological information of a person to be measured; an image-shooting device; and an information-processing device, the fraudulent-measurement determination method having:
a first step of displaying indication identification information on display means and sending device identification information to the information-processing device;
a second step of the image-shooting device shooting an image of the indication identification information displayed on the display means;
a third step of the information-processing device receiving image information acquired by the image-shooting device shooting the image and the device identification information sent from the biological information measuring device; and
a fourth step of the information-processing device determining whether the person to be measured is measuring the biological information using the biological information measuring device an image of which has been shot by the image-shooting device or not based on the indication identification information indicated by the image information and the device identification information sent from the biological information measuring device.

14. A fraudulent-measurement determination program for a biological information measuring system comprising: a biological information measuring device for measuring biological information of a person to be measured; an image-shooting device; and an information-processing device, the fraudulent-measurement determination program being for causing a computer to execute:
a first step of displaying indication identification information on display means and sending device identification information to the information-processing device;
a second step of the image-shooting device shooting an image of the indication identification information displayed on the display means;
a third step of the information-processing device receiving image information acquired by the image-shooting device shooting the image and the device identification information sent from the biological information measuring device; and
a fourth step of the information-processing device determining whether the person to be measured is measuring the biological information using the biological information measuring device an image of which has been shot by the image-shooting device or not based on the indication identification information indicated by the image information and the device identification information sent from the biological information measuring device.

15. A computer-readable non-transitory storage medium holding the fraudulent-measurement determination program according to claim 14.
